(19) **Europäisches Patentamt / European Patent Office / Office européen des brevets**

(11)  **EP 1 978 026 B1**

(12)  # EUROPEAN PATENT SPECIFICATION

(45) Date of publication and mention
of the grant of the patent:
**16.03.2011  Bulletin 2011/11**

(51) Int Cl.:
***C07K 7/08*** *(2006.01)*      ***A61K 38/10*** *(2006.01)*

(21) Application number: **06841747.6**

(86) International application number:
**PCT/ES2006/000695**

(22) Date of filing: **19.12.2006**

(87) International publication number:
**WO 2007/074188 (05.07.2007 Gazette 2007/27)**

(54)  **T-HELPER ANTIGENIC DETERMINANT (THD) PEPTIDES**

T-HELPER ANTIGENIC DETERMINANT (THD) PEPTIDE

PEPTIDES DETERMINANTS ANTIGENIQUES T AUXILIAIRES (DTA)

(84) Designated Contracting States:
**AT BE BG CH CY CZ DE DK EE ES FI FR GB GR
HU IE IS IT LI LT LU LV MC NL PL PT RO SE SI
SK TR**
Designated Extension States:
**AL BA HR MK RS**

(30) Priority:  **23.12.2005  ES 200503169**

(43) Date of publication of application:
**08.10.2008  Bulletin 2008/41**

(73) Proprietor: **Proyecto de Biomedicina Cima, S.L.
31008 Pamplona - Navarra (ES)**

(72) Inventors:
• **BORRAS  CUESTA, Francisco
31008 Pamplona (Navarra) (ES)**
• **LASARTE  SAGASTIBELZA, Juan José
31008 Pamplona ( Navarra) (ES)**
• **RUIZ EGOZCUE, Marta
31008 Pamplona (Navarra) (ES)**
• **SAROBE  UGARRIZA, Pablo
31008 Pamplona (Navarra) (ES)**

(74) Representative: **Ungria Lopez, Javier
Avda. Ramón y Cajal, 78
28043 Madrid (ES)**

(56) References cited:
**WO-A1-95/07707       WO-A1-98/32456
WO-A1-99/61916        US-A1- 2004 170 644
US-A1- 2004 236 514**

• **RUIZ ET AL: "Engineered promiscuous T helper peptides for the induction of immune responses" MOLECULAR IMMUNOLOGY, PERGAMON, GB, vol. 44, no. 9, 8 December 2006 (2006-12-08), pages 2205-2212, XP005834038 ISSN: 0161-5890**
• **ALEXANDER J. ET AL.: 'Development of high potency universal DR-restricted helper epitopes by modification of high affinity DR-blocking peptides' IMMUNITY vol. 1, no. 9, December 1994, pages 751 - 761, XP000673954**
• **BORRAS-CUESTA F. ET AL.: 'Specific and general HLA-DR binding motifs: comparison of algorithms' HUMAN IMMUNOLOGY vol. 61, no. 3, 2000, pages 266 - 278, XP003015204**

**Description**

**FIELD OF THE INVENTION**

The present invention is within the field of the determination of antigenic peptides, capable of stimulating T-helper responses.

**PRIOR ART**

T-helper lymphocytes (ThL) perform various important functions in immunity to pathogens. In first place, the induction of an effective effector immune response, either a humoral response or a cytotoxic cellular response, requires the activation of ThL, and more specifically of specific subpopulations of ThL (Th1, Th2, Th0). Secondly, the ThL can also act directly as effector cells, an activity mediated by direct cell contact or by the release of lymphokines (IFN-γ, TNF-α, etc.). Therefore, the stimulation of T-helper (Th) responses constitutes a very relevant aspect for the development of vaccines.

It is well known that to achieve a stimulating effect, the ThL recognize, through specific receptors (CTR) situated on its surface, complexes formed between Class II MHC molecules and antigenic peptides. These peptides which bind to the Class II MHC molecules, also known as Th epitopes or Th antigenic determinants (Thd), typically have sizes between 11 and 22 amino acids, and more frequently between 13 and 16 amino acids.

In recent years, vaccines based on epitopes have awoken considerable interest as a possible tool in the development of new vaccines and immunotherapeutic strategies. A careful selection of epitopes for B and T cells should permit directing the immune responses towards conserved epitopes of certain pathogens, characterized by great sequence variability (e.g. malaria, hepatitis C virus, HIV, etc.).

Furthermore, vaccines based on epitopes offer the opportunity of including chimeric Thd which have been manufactured to modulate their stimulating potency, either increasing their binding capacity with the MHC molecules of the main histocompatibility complex, or modifying the contact residues with the TCR receptors of T cells, or modifying both characteristics. Due to the chimeric nature of these peptides, there are very few probabilities that their sequence is contained on own antigens, for which reason, if after their use, their antibodies were induced against the peptides, there would be very little probability of inducing undesired responses against own antigens.

The prediction and selection of the appropriate epitopes comes up, however, against an important obstacle: the great number of polymorphisms existing between the MHC molecules, which very particularly affect the binding regions to the epitope and ThL recognition. This polymorphism is produced as a result of the polygenic character of MHC histocompatibility and the great number of allelic variants existing for each one of these genetic loci. Thus, for example, human Class II MHC comprises 3 pairs of genes (each pair with its α and β chain), called HLA-DR, HLA-DP and HLA-DQ, which give rise to 4 basic types of Class II HLA molecules. A general review can be found in the manual: Immunobiology - The immune system in health and disease; Janeway CA Jr and Travers P Eds.; Current Biology Ltd / Garland Publishing Inc., London, 1997 3rd Ed. This polymorphism gives rise to the expression of many different MHC molecules, each of them with different ranges of specificity for the binding of epitopes (MHC restriction).

Although the specific allele polymorphic residues which surround the binding groove to the epitope give the MHC molecule the capacity to bind to a certain set of peptides, there are several cases wherein a same peptide can bind to one more than one allelic form of the MHC molecule. This has particularly been verified for HLA-DR molecules, where various allelic forms HLA-DR may recognise similar peptide motifs, at the same time as it has been verified that certain peptides are recognized by different HLA-DR molecules. This has led to the concept that certain peptides could represent promiscuous or universal epitopes.

Thus, the use of different algorithms has permitted defining various motifs useful for the selection of epitopes, having identified some universal epitopes recognized by a good number of isoforms of the HLA molecules, and more particularly of HLA-DR (WO95/07707; Alexander J et al. Immunity, 1994, 1:751-761; WO98/32456).

This last type of more promiscuous peptide may be of great use in inducing humoral and cellular responses in a great diversity of healthy individuals, which would avoid having to choose special peptides depending on the HLA-DR of said individuals.

Although a set of these promiscuous PADRE peptides is already available (Alexander J et al. Immunity, 1994, 1:751-761), it continues to be of interest to identify new promiscuous chimeric peptides. This is due to despite that fact that all these peptides share being recognized by several HLA-DR, some peptides may be better than others for a specific HLA-DR. Consequently, it would be of great use to have a wider battery of promiscuous peptides to thus better cover the induction of responses compared with the totality of the HLA-DR. Furthermore, it is also desirable to identify peptides which are bound and can be recognized in the context of the other HLA-DP and HLA-DQ isotopes. This would permit generating vaccines and immotherapeutic products for a wider spectrum of persons.

## DETAILED EXPLANATION OF THE INVENTION

With the purpose of identifying new chimeric peptides which had the potential of binding strongly to different HLA-DR molecules, and in consequence, being capable of providing help for the induction of antibodies and also cytotoxic T responses, a set of peptides of 13 amino acids was synthesized. Formulas or templates of sequences were established for this, devised taking a motif of 8 amino acids described by the inventors themselves as starting reference (Borrás-Cuesta F. et al.; Specific and general HLA-DR binding motifs: comparison algorithms; Human Immunol., 2000; 61: 266-278).

Firstly, peptides were synthesized whose sequence adapted to the formula:

$$\text{I) } a_1\text{-}a_2\text{-}Y\text{-}R\text{-}a_5\text{-}M\text{-}a_7\text{-}R\text{-}a_9\text{-}R\text{-}A\text{-}A\text{-}A;$$

where Y is Tyr; R is Arg; M is Met; A is Ala; $a_1$ is Phe or Tyr; $a_2$ is Lys or Arg; $a_5$, $a_7$ and $a_9$ are any of the 20 natural amino acids.

In all cases, a tyrosine was used as primary anchor in the third residue (first residue of the aforementioned motif). Furthermore, to reach the typical length of 13 amino acids in most of the Thd (Chicz R.M. et al.; Predominant naturally processed peptides bound to HLA-DR1 are derived from MHC-related molecules and are heterogeneous in size; Nature, 1992; 358: 764-768), three alanines were added to the nucleus of 8 amino acids at their C-terminal end and another two amino acids at their N-terminal ends: an aromatic amino acid (phenylalanine or tyrosine) in the first residue and an amino acid with positive charge (lysine or arginine) in the second residue. The use of phenylalanine or tyrosine in the first residue provides an additional anchoring point.

Furthermore, the amino acids which occupy positions 4, 6, 8 and 10 of the peptides were fixed in said formula.

Other formulas for the synthesis and evaluation of peptides were established from the first formula, wherein the possibility was left open of varying two of the four amino acids fixed in aforementioned positions 4, 6, 8 and 10. The formulas tested were the following:

$$\text{II) } a_1\text{-}a_2\text{-}Y\text{-}R\text{-}a_5\text{-}M\text{-}a_7\text{-}a_8\text{-}a_9\text{-}a_{10}\text{-}A\text{-}A\text{-}A;$$

$$\text{III) } a_1\text{-}a_2\text{-}Y\text{-}a_4\text{-}a_5\text{-}M\text{-}a_7\text{-}a_8\text{-}a_9\text{-}R\text{-}A\text{-}A\text{-}A;$$

$$\text{IV) } a_1\text{-}a_2\text{-}Y\text{-}R\text{-}a_5\text{-}a_6\text{-}a_7\text{-}a_8\text{-}a_9\text{-}R\text{-}A\text{-}A\text{-}A;$$

where Y is Tyr; R is Arg; M is Met; A is Ala; $a_1$ is Phe or Tyr; $a_2$ is Lys or Arg; $a_4$ is any of the 20 natural amino acids other than Arg; $a_5$, $a_7$ and $a_9$ are any of the 20 natural amino acids; $a_6$ is any of the 20 natural amino acids other than Met; $a_8$ is any of the 20 natural amino acids other than Arg; and $a_{10}$ is any of the 20 natural amino acids other than Arg.

A peptide of the following sequence was also synthesized:

$$\text{V) SEQ. ID. NO: 21,}$$

wherein the amino acids were varied in 3 of the initially fixed positions, keeping methionine in position 6.

For comparative purposes, short peptides of 8 and 9 amino acids were also synthesized which also had tyrosine as primary anchor and in the majority of the remaining positions of the nucleus, amino acids that favour binding to HLA-DR.

Once synthesized, its capacity of binding strongly to different allelic forms of the HLA-DR, molecule was evaluated, with the result that the majority were capable of strongly binding to at least one of the allelic forms.

A general sequence was obtained from the above. Thus, in a first embodiment, the present invention relates to a chimeric peptide with capacity to bind to at least one allelic form of the HLA-DR molecule, characterized in that its sequence of amino acids adapts to a formula selected from:

$$\text{a) } a_1\text{-}a_2\text{-}Y\text{-}a_4\text{-}a_5\text{-}a_6\text{-}a_7\text{-}a_8\text{-}a_9\text{-}a_{10}\text{-}A\text{-}A\text{-}A; \text{ and}$$
$$\text{b) SEQ. ID. NO: 21;}$$

where Y is Tyr; A is Ala; $a_1$ is Phe or Tyr; $a_2$ is Lys or Arg; $a_4$ is Arg, except when $a_6$ and $a_{10}$ are Met and Arg, respectively, where $a_4$ can be any of the 20 natural amino acids; $a_5$, $a_7$ and $a_9$ are any of the 20 natural amino acids; $a_6$ is Met except when $a_4$ and $a_{10}$ are Arg, case wherein $a_6$ is any of the 20 natural amino acids; $a_8$ is Arg, except when $a_4$ is Arg, Tyr or His, $a_6$ is Met or Val and $a_{10}$ is Met, His or Arg, case wherein $a_8$ is any of the 20 natural amino acids; and $a_{10}$ is Arg, except when $a_4$ is Arg or His and $a_6$ is Met, case wherein $a_{10}$ is any of the 20 natural amino acids.

Therefore, a second aspect of the present invention relates to a chimeric peptide with capacity to bind to at least one allelic form of the HLA-DR molecule whose sequence of amino acids adapts to one of the previously defined formulas I), II), III), and IV). Hereinafter, we refer to this as "chimeric peptide of the invention" or "peptide of the invention". In a particular embodiment said HLA-DR allelic form corresponds to the HLA-DR1, HLA-DR2, HLA-DR3, HLA-DR4, HLA-DR7, HLA-DR8 or HLA-DR11 serotype.

In a particular embodiment, the chimeric peptide of the invention strongly binds to at least 2 allelic forms of HLA-DR of different serotype, and preferably 3, 4, 5, 6 or even 7 of these allelic forms.

In some cases, the chimeric peptide of the invention can also bind to other isotopes of Class II HLA molecules, for example HLA-DP or HLA-DQ. In a particular embodiment, they also bind to some allelic forms of HLA-DQ.

In a preferred embodiment, the chimeric peptide of the invention behaves as a Th antigenic epitope or determinant (Thd). The terms Th or Thd determinant are indiscriminately used and mean that said peptide, bound to the HLA molecule, is recognized by Th lymphocytes, and is capable of inducing the activation of said Th lymphocytes or T-helper cells (Th response). This activation is evidenced by its capacity for inducing the proliferation of Th lymphocytes and to induce the production of specific lymphokines of these Th lymphocytes, such as IL-4, IFN-$\gamma$ or TNF-$\alpha$. The Th response induced can be a Th1 or Th2 response, or a mixed Th0 response. This capacity of acting as Thd is possible in the context of at least one of the forms of HLA-DR, HLA-DP or HLA-DQ indicated.

Preferably, the chimeric peptide of the invention is also capable of inducing an effective humoral or cytotoxic T response. In an embodiment said response is a CT response.

In a particular embodiment, the chimeric peptide of the invention is a peptide of sequence SEQ. ID. NO: 1, SEQ. ID. NO: 4, SEQ. ID. NO: 5, SEQ. ID. NO: 6, SEQ. ID. NO: 7, SEQ. ID. NO: 10, SEQ. ID. NO: 11, SEQ. ID. NO: 12, SEQ. ID. NO: 13, SEQ. ID. NO: 14, SEQ. ID. NO: 15, SEQ. ID. NO: 16, SEQ. ID. NO: 17, SEQ. ID. NO: 20 or SEQ. ID. NO: 22.

The chimeric peptides of the invention can be obtained by conventional methods, for example, by solid phase chemical synthesis techniques; purification by high performance liquid chromatography (HPLC); and, if desired, they can be analysed using conventional techniques, for example, by sequencing or mass spectrometry, amino acid analysis, nuclear magnetic resonance, etc. Alternatively, the peptides of the invention can also be obtained via recombinant DNA technology.

The chimeric peptides of the invention could be used for administration to a subject (a man, a woman or any other mammal) with immunoprophylactic or immunotherapeutic purposes. Therefore, in another aspect, the invention also relates to a pharmaceutical composition which contains a chimeric peptide of the invention (or a plurality thereof) and a pharmaceutically acceptable excipient.

In a particular embodiment, a chimeric peptide of the invention (or a plurality thereof) can be administered in an immunostimulating combination together with another or other immunogens different from the chimeric peptides of the invention. This combination can be presented in the form of a single pharmaceutical composition or separate pharmaceutical compositions for combined administration, by a simultaneous or sequential administration, by the same administration route or by different routes. Thus, the present invention also relates to a pharmaceutical composition characterized in that it comprises a chimeric peptide of the invention and another immunogen.

The term "immunogen" relates to a molecule which is cable of inducing a specific immunological response to said immunogen (humoral: production of antibodies; or cellular: activation of Th lymphocytes, activation of CT lymphocytes, etc.). Due to its chemical nature, the immunogen can be almost any molecule: for example, polypeptides, lipopeptides, oligosaccharides, polysaccharides, nucleic acids, lipids or other chemical compounds as drugs. By its origin, said immunogen may come, for example, from a pathogen (virus, bacteria, fungus, parasite, etc.), of a tumour cell, of synthesis (drugs or other synthesis compounds) or of any other origin (for example, allergens). In some cases, said immunogen is a proteic antigenic determinant, for example a Th antigenic determinant or a CT antigenic determinant.

In a more particular embodiment, the pharmaceutical composition of the invention contains a cytotoxic T determinant (CTd) and a chimeric peptide of the invention (or a plurality thereof) which acts as T-helper determinant (Thd).

When the pharmaceutical composition contains a chimeric peptide of the invention and another or other immunogens, these may be presented as separate molecules or in conjugated form, for example, by covalent bonds. The conjugation may be performed by various conventional methods which are described, for example, in: "The current protocols in protein chemistry", published by John Wiley & Sons (periodically updated; Last updated 1 May 2005); "Immobilized affinity ligand Techniques", GT Hermanson, AK Mallia and PK Smith, Academic Press, Inc. San Diego, CA, 1992; EP0876398; among others.

The pharmaceutical composition which comprises a chimeric peptide of the invention may additionally contain carriers, excipients and other known pharmaceutically acceptable ingredients.

Still in another additional aspect, the invention relates to the use of a chimeric peptide of the invention (or a plurality thereof) in the preparation of an immunostimulating pharmaceutical composition. This pharmaceutical composition may be used to induce a specific immune response to an immunogen administered in combination with a chimeric peptide, within the same composition or in separate compositions as has been previously described. In this way, the chimeric peptide of the invention is used to induce a Th response (activation of Th lymphocytes) in a subject administered the pharmaceutical composition. Said response can be a Th 1 or Th2 response or a mixed Th0 response.

In a particular embodiment, this Th response cooperates in the activation of B lymphocytes, so that the pharmaceutical composition with the chimeric peptide is useful for inducing a humoral immune response.

In another embodiment, the Th response collaborates in the activation of CT lymphocytes, so that the pharmaceutical composition is useful for inducing a cytotoxic T cell response (CT).

Additionally, the immunostimulating pharmaceutical composition with the chimeric peptide of the invention may have other uses, such as, for example, the *in vitro* treatment or pre-conditioning of dendritic cells with therapeutic purposes.

In consequence, the immunostimulating pharmaceutical composition which contains a chimeric peptide of the invention is useful for the treatment and prophylaxis of an infectious (bacterial, viral, fungal or parasitic), tumoral or allergic disease.

The immunostimulating pharmaceutical composition of the invention can be applied to any animal or human subject: e.g. mammals (human or otherwise), birds and similar. For this, any suitable route of administration can be used in accordance with the known conventional methods of the state of the art. A review of the different pharmaceutical forms of administration of drugs and excipients necessary for their production can be found, for example, in "Tecnologia farmaceutica", by J.L. Vila Jato, 1997 Vols I and II, Ed. Synthesis, Madrid; or in "Handbook of pharmaceutical manufacturing formulations", by S.K. Niazi, 2004 Vols I a VI, CRC Press, Boca Raton. In a particular embodiment, the pharmaceutical composition is administered by parenteral route (e.g. intravenous, subcutaneous, intramuscular, intraperitoneal), transdermal, mucosal or similar.

## BRIEF DESCRIPTION OF THE FIGURES

Figure 1. Binding capacity of chimeric peptides to different HLA-DR molecules. It is

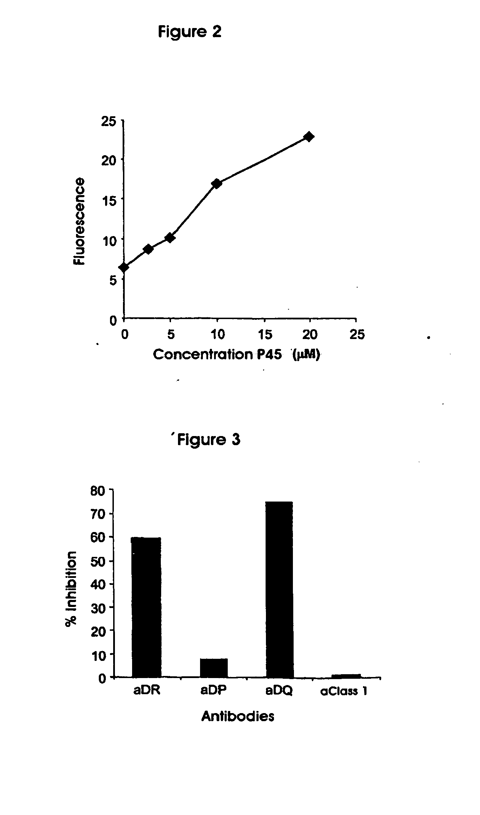

Figure 2. expressed as a percentage of relative binding (%$B_R$), in terms relative to the binding of the non biotinylated HA control peptide (306-320): APKYVKQNTLKLATG. The density of the grids represent an increasing percentage order, in accordance with the key at the foot of the figure. Binding of the biotinylated P45 peptide to the HLA-DR4 cell line. HLA-DR4 cells were incubated with different concentrations of biotinylated peptide and their fluorescence was measured (expressed as arbitrary units of fluorescence), which is directly proportional to the concentration of the biotinylated P45 peptides that have bound.

Figure 3. Percentage of inhibition of binding of the biotinylated P45 peptide to cells which express HLA-DR4, in the presence of specific anti-HLA antibodies: aDR, anti-HLA-DR; aDP, anti-HLA-DP; aDQ, anti-HLA-DQ; and Class I anti-HLA.

Figure 4. Induction of T-helper responses in transgenic HLA-DR4 mice immunized with different peptides (50 nanomoles): p37, p45, p61, p62 and PADRE. The responses to each peptide were evaluated after 15 days: lymphocytic proliferation, production of IFN-γ, and production of IL-4.

Figure 5. Induction of cytotoxic T responses in transgenic HLA-DR4 mice immunized with a CTd peptide [50 nanomoles of OVA (257-264)] alone or together with one of the peptides to test as Thd: p37, p45, p61, p62 or PADRE. The assays were repeated with different concentrations of peptides to test: A) 50 nanomoles; B) 5 nanomoles; C) 0.5 nanomoles.

## EMBODIMENT OF THE INVENTION

### Example 1. Peptide synthesis

The peptides for the assays of binding to the HLA molecules and induction of T-helper (Th) and cytotoxic T (CT) responses were manually synthesized by the Merrifield solid phase method, using the Fmoc technology [(Merrifield RB; Solid phase synthesis. I. J Am Chem Soc, 1963; 85:2149); (Atherton E Procedures for solid phase synthesis. J Chem Soc Perkin Trans, 1989; 1:538)]. Both the peptides to test and the peptides used as control were synthesized using this same method (Table 1).

**Table 1. Peptides synthesized for the assays of binding to HLA molecules and induction of T-helper (Th) and cytotoxic (CT) responses.**

| Name | Sequence | SEQ. ID. NO: |
|------|----------|--------------|
| p45 | FKYRMMMRMRAAA | 1 |
| p44 | YRMMMRMRA | 2 |
| p43 | YRMMMRMR | 3 |

(continued)

| Name | Sequence | SEQ. ID. NO: |
|---|---|---|
| p61 | FRYRMMMRMRAAA | 4 |
| p62 | YRYRMMMRMRAAA | 5 |
| p53 | FKYRWMMRWRAAA | 6 |
| p52 | FKYRRMMRKRAAA | 7 |
| p41 | YRAMRAMRA | 8 |
| p40 | YRAMRAMR | 9 |
| p46 | FKYRMMMAPMAAA | 10 |
| p42 | FKYRAMRAMRAAA | 11 |
| p49 | FKYRAMRCMRAAA | 12 |
| p50 | FKYRAMRRRRAAA | 13 |
| p51 | FKYRRMRRRRAAA | 14 |
| p57 | FKYRWMRAMRAAA | 15 |
| p37 | FKYRQMMAPHAAA | 16 |
| p48 | FKYRAMRRRHAAA | 17 |
| p39 | YRQMMAPHA | 18 |
| p47 | YRAMRRRHA | 19 |
| p58 | FKYYAMRCMRAAA | 20 |
| p56 | FKYHQMMAPHAAA | 21 |
| p60 | FKYRWVRALRAAA | 22 |
| PADRE | AKFVAAWTLKAAA | 23 |
| HA(306-320) | APKYVKQNTLKLATG | 24 |
| OVA(257-264) | SIINFEKL | 25 |

Biotinylated peptides were also used for some assays: the HA (306-320) (APKYVKQNTLKLATG) peptide of the hemaglutinine of the Flu virus and p45. These peptides were synthesized manually and were conjugated with biotin (EZ-Link Sulfo-NHS-LC-Biotin; Pierce Biotechnology, Inc, Rockford, USA). For this, once the peptide synthesis had concluded, this remained bound to the resin and 10 washes were performed with a DMF-water mixture (7.5:2.5) to prepare the resin to this new solvent. Biotin dissolved in this solvent was added, in proportion (1:1) with the milliequivalents of the initial resin. The mixture was allowed to react for one and a half hours. Next, the resin was washed 20 times with DMF, and the reaction process was repeated up to 3 times. To check that the peptide was biotinylated, the Kaiser test was performed (Kaiser, 1970; Color test for detection of free terminal amino groups in the solid-phase synthesis of peptides. Anal Biochem. 1970; 34:595-598). The resin was cut, liophilised and analysed by HPLC as in the previous section. [(Merrifield RB; Solid phase synthesis. I. J Am Chem Soc, 1963; 85:2149); (Atherton E Procedures for solid phase synthesis. J Chem Soc Perkin Trans, 1989; 1:538)].

The PADRE peptide was synthesized for comparative purposes. This is a peptide similar to another previously developed (Alexander J et al. Immunity, 1994, 1:751-761) with the purpose of inducing T-helper responses in a wide variety of HLA-DR molecules. This PADRE peptide was differentiated from the previously described peptide in that it was synthesized with the amino acid phenylalanine instead of the original cyclohexylalanine.

## Example 2. Assays of binding of the peptides to different HLA molecules.

*Binding to HLA-DR molecules*

The binding of the peptides was measured as described by Busch et al. (Busch R, Rothbard J: Degenerate binding of immunogenic peptides to HLA-DR proteins on B cell surfaces. Int Immunol, 1990; 144:1849).
In the experiments of the present invention, the following lines of B lymphocytes were used transformed by Epstein-Barr virus (EBV-BLCL); each one of them homozygotic for different HLA-DR molecules:

| Line | ECACC No. | Molecular typing | Serological typing |
|---|---|---|---|
| HOM-2 | 88052005 | DRB1*0101 | DR1 |
| WT8 | 88052017 | DRB1*1501 | DR2 |
| RSH | 88052021 | DRB1*0302/DRB3*0101 | DR3 |

(continued)

| Line | ECACC No. | Molecular typing | Serological typing |
|------|-----------|------------------|-------------------|
| BOLETH | 88052031 | DRB1*0401/DRB4*0101 | DR4 |
| MOU | 88052050 | DRB1*0701/DRB4*0101 | DR7 |
| OLGA | 88052100 | DRB1*0802 | DR8 |
| SWEIG | 88052037 | DRB1*1101/DRB3*0202 | DR11 |

All the cell lines were obtained from the European Collection of Animal Cell Cultures (ECACC, PHLS, Salisbury, UK).

Briefly, B lymphocytes with different HLA-DR molecules (at $2.5 \times 10^5$ cells/well) were coincubated throughout the night with biotinylated HA(306-320) (10 $\mu$M) and non-biotinylated HA(306-320) (100 $\mu$M)on the one side, or with biotinylated HA(306-320) (10 $\mu$M) and the peptide to test (100 $\mu$M) on the other. The incubation was carried out in complete MC medium (RPMI 1640 with 10% calf foetal serum, 2mM of glutamine, 100 U/ml of penicillin, 100 $\mu$g/ml of streptomycin, $5 \times 10^{-5}$ M of 2 $\beta$-mercaptoethanol, and 0.5% (v/v) of sodium pyruvate). On the next day, 2 washes were carried with 200 $\mu$l of FACS medium (2.5% PBS of calf foetal serum); 5 $\mu$g/ml of streptavidin-fluorescein (Pierce) in 100 $\mu$l of FACS medium and they were incubated at 4 °C for 30 minutes. Next, 2 washes were performed and the cells were resuspended in 200 $\mu$l of FACS medium.

The fluorescence of the cell surface was measured by flow cytometry in a FACScan analyser (Becton Dickinson Immunocytochemistry System, Mountain, USA). The mean fluorescence of 5,000 labelled cells was measured. A fluorescence signal was obtained proportional to the number of HLA-DR molecules exposed on the outside of the cell.

The following formula was used to quantify the binding capacity of each peptide (% Binding$_{peptide}$):

$$\% \text{ Binding}_{peptide} = 100 \times ((F_{peptide} - F_{blnk}) / (F_{ctrl.biot} - F_{blnk}))$$

where $F_{peptide}$ is the fluorescence measured by the peptide to test $F_{blnk}$ is the fluorescence measured without added peptide (blank); and $F_{ctrl.biot}$ is the fluorescence measured for the biotinylated control peptide [HA(306-320)].

In this way, the binding percentage was calculated using the non-biotinylated control peptide [HA(306-320)] as test peptide(%Binding$_{ctrl}$):

$$\% \text{Binding}_{ctrl} = 100 \times ((F_{ctrl.nobiot.} - F_{blnk}) / (F_{ctrl.biot.} - F_{blnk}))$$

HA (306-320) was used as reference control, instead of the CPKYVKQNTLKLATG peptide as previously described (Rothbard JB; Degenerate binding of immmunogenic peptides. Int Immunol 1990; 2:443-451), in order to prevent the formation of potential disulfur bridges via cysteine - $NH_2$ terminal.

The relative binding percentages (% B$_R$) was also calculated according to the following formula:

$$\% \text{ B}_R = 100 \times (\% \text{ Binding}_{peptide} / \% \text{ Binding}_{ctrl})$$

where % Binding$_{peptide}$ is the binding percentage of the peptide to test; and where % Binding$_{ctrl}$ is the binding percentage of the non-biotinylated of the HA(306-320) control peptide.

All the assays were performed in triplicate. The variation in the fluorescence intensities of the triplicates was always in the 5-10% range.

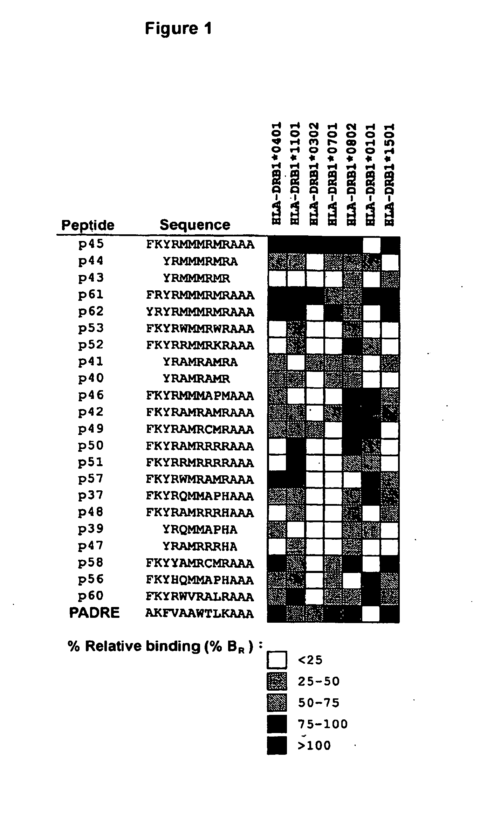

In this was it was possible to obtain an evaluation of the binding capacity of the different peptides to HLA-DR1, HLA-DR2, HLA-DR3, HLA-DR4, HLA-DR7, HLA-DR8, HLA-DR11 molecules, expressed in terms relative to the binding of the non-biotinylated HA peptide: APKYVKQNTLKLATG (Fig. 1). From figure 1 it can be concluded that:

- Most peptides bind with good affinity to at least two HLA-DR molecules;
- in particular, the p45, p61 and p62 peptides bind with good affinity to almost all the HLA-DR molecules studied.

The p45, p61 and p62 peptides exhibited a binding capacity comparable to or even greater than the PADRE peptide tested.

*Binding of p45 to HLA-DR, HLA-DP and HLA-DQ molecules*

p45 was fairly insoluble. In order to better characterize its binding capacity, and to reject a possible toxic effect of the peptide, it was decided to perform some complementary tests using biotinylated p45.

In first place, tests were performed for binding to HLA-DR in the different cell lines, incubated with biotinylated p45 at different concentrations. To avoid the possible crystallization of the peptide, this was solubilized with the aid of a sonicator. The fluorescence of the cell surface was measured by flow cytometry in a FACScan analyser as seen in example 2, although there was no competition with the non-biotinylated p45 peptide. Figure 2 shows the fluorescence measured in the binding tests in the line expressed by H-LA=DR4. As can be verified, the peptide binding is dose-dependent in the range of concentrations tested.

In second place, the HLA-DR4 cell line was incubated in the presence of the biotinylated P45 peptides and antibodies selected due to their specificity to HLA-DR, HLA-DP, HLA-DQ and Class I HLA respectively (figure 3).

In a 96-well plate with U-shaped bottom, the HLA-DR4 cell line was seeded (already defined); ($2 \times 10^5$ per well), also adding biotinylated P45 peptides (10 $\mu$M), alone or together with supernatant of the hybridomas: L243 anti-HLA-DR (ATCC Ref: HB-55), or W6/32 anti-Class I (ATCC Ref: HB-95) or the antibodies 33.1 anti-HLA-DQ or anti-HLA-DP B7/21, which were provided by Dr. Ghislaine Sterkers. All were diluted to (1/500) in a final volume of 100 $\mu$l of RPMI with 2.5 % FBS. The next day, 2 washes were performed with 200 $\mu$l of FACS medium, 5 $\mu$g/ml of streptavidin-fluorescein conjugate (Pierce) in 100 $\mu$l of FACS, and they were they were incubated at 4 °C for 30 minutes. Next, 2 washes were performed and the cells were resuspended in 200 $\mu$l of FACS medium. The fluorescence of the cell surface was measured by flow cytometry in a FACScan analyser. The mean fluorescence of 5,000 labelled cells was measured. A fluorescence signal was obtained proportional to the number of HLA-DR molecules exposed on the outside of the cell.

The following formula was used to quantify the decrease in binding capacity on adding the antibodies:

$$\% \text{ Inhibition} = 100 \text{ x } ((F_{p45+aHLA} - F_{blnk}) / (F_{p45} - F_{blnk}))$$

where $F_{blnk}$ is the fluorescence measured when the cells were cultured without adding peptide or antibodies (blank), $F_{p45}$ is the fluorescence measured when it was incubated with the biotinylated P45 peptides alone, and $F_{p45+aHLA}$ is the fluorescence measured when it was measured with the biotinylated p45 together with the corresponding HLA antibodies.

All the assays were performed in triplicate. The variation in the fluorescence intensities of the triplicates was always in the 5-10% range.

As can be seen in Figure 3, incubation with anti-HLA-DR or anti-HLA-DQ antibodies produces strong inhibition of the binding, which indicates that p45 binds both to HLA-DR and HLA-DQ, but not to HLA-DP.

In this way, these tests were repeated on the HLA-DR1, HLA-DR3, HLA-DR7, HLA-DR8, HLA-DR11 cell lines. The inhibition percentages obtained are set down in Table 2. As can be observed, when the cells were incubated with biotinylated p45 in the presence of anti-HLA-DR or anti-HLA-DQ, a strong inhibition occurs in all cases, which indicates that the biotinylated p45 has a high capacity of binding to HLA-DR and to HLA-DQ in all the cell lines.

The biotinylated P45 peptides bound to HLA-DR1, although non-biotinylated p45 does not bind in detectable manner to this HLA molecule (see Fig 1). This phenomenon of greater binding of the biotinylated peptide is also observed in the biotinylated HA peptide (306-320) with respect to non-biotinylated HA(306-320). This could indicate that biotin stabilizes the binding to the HLA molecule in additional form or that it increases the sensitivity of the detection of the binding with respect to the measurement for competition with the non-biotinylated peptide. The other peptides in the study were non-biotinylated, which means there is the possibility that they may also bind to HLA-DQ.

**Table 2. Inhibition (%) of the binding of biotinylated p45 to the HLA molecules of different cell lines.**

| Cell line | Serotype HLA-DR | Molecular typing HLA-DR - HLA-DQ | Percentage of inhibition (%) | | | |
|---|---|---|---|---|---|---|
| | | | aDR | aDP | aDQ | aCII |
| HOM2 | DR1 | DRB1*0101 - DQB1*0501 | 78 | 23 | 75 | 13 |
| RSH | DR3 | DRB1*0302 - DQB1*0402 | 86 | 28 | 98 | 6 |
| BOLETH | DR4 | DRB1*0401 - DQB1*0302 | 59 | 7 | 75 | 1 |

(continued)

| Cell line | Serotype HLA-DR | Molecular typing HLA-DR - HLA-DQ | Percentage of inhibition (%) | | | |
|---|---|---|---|---|---|---|
| | | | aDR | aDP | aDQ | aCII |
| MOU | DR7 | Drub1*0701 - DQB1*0201 | 71 | 13 | 98 | 0 |
| OLGA | DR8 | DRB1*0802 - DQB1*0402 | 92 | 4 | 88 | 5 |
| SWEIG | DR11 | DRB1*11011 - Dub1*0301 | 89 | 0 | 83 | 0 |

NB: The degree of binding to the different molecules was measured for competition with antibodies (aDR: anti-HLA-DR; aDP: anti-HLA-DP; aDQ: anti-HLA-DQ; aC1I: anti-Class I).

**Example 3. Induction of T-helper responses (Th).**

In order to check if the synthesized peptides had the capacity of inducing Th responses *in vivo,* transgenic mice were immunized for the HLA-DR4 molecule with some of the peptide which had demonstrated binding capacity with various HLA-DR molecules. For this, p37, p45, p61 and p62 were chosen, also using the PADRE peptide as control. All these peptides showed binding capacity to several HLA-DR molecules, whilst they showed different degrees of binding to HLA-DR4. The Th inducing capacity was evaluated measuring the peptide's capacity of inducing cell proliferation and of inducing the production of IFN-$\gamma$ and IL4 in lymphocytes extracted from the immunized mice.

*Immunization*

HLA-DR4 transgenic female mice obtained from Taconic were used (Germantown, NY, USA), which were maintained in conditions free from pathogens and treated following the standards of our institution.

For the induction of Th responses, groups of 3 mice were immunized (4-6 weeks old) with 200 $\mu$l of a 1:1 emulsion of complete Freund's adjuvant and saline solution which contained 50 nanomoles of the corresponding peptide. The immunized animals were sacrificed two weeks after immunization and the popliteal, inguinal and periaortic lymph nodes were extracted. The nodes were homogenized with a syringe and were washed three times in a washing medium (RPMI 1640 medium) at 4 °C. Next, $5 \times 10^7$ cells/ml were pulsed in MC during 2 hours at 37 °C with 10 $\mu$M of the corresponding peptide.

Then, they were centrifuged and resuspended and $2 \times 10^6$ cells/ml were cultured in a volume of 2 ml, in a 24 well plate, in an oven at 37 °C with 5% $CO_2$. Seven days later, the cells were washed and $5 \times 10^5$ T cells were cultured per well with $2 \times 10^5$ cells of syngenic spleen per well, treated with mitomicyn-C, in the absence or presence of the corresponding antigen. 50 $\mu$l of the supernatant were collected to measure IFN-$\gamma$ and IL-4 as in the previous section. The cell proliferation was measured.

*Measurement of cell proliferation*

After 48 hours in culture, the cells were pulsed with 0.5 $\mu$Ci of tritiated thymidine during 18 hours, they were harvested and the incorporation of thymidine was determined in a scintillation counter (Top-count; Packard, Meridan, CT, USA).

*Measurement of IFN-$\gamma$ and IL-4 production*

The quantities of IFN-$\gamma$ and IL-4 were measured using commercial ELISA (OPTEIA Mouse IFN-$\gamma$ Set, Pharmingen, San Diego, USA and OPTEIA Mouse IL-4 Set, Pharmingen, San Diego, USA) in accordance with the manufacturer's instructions. The results were expressed as pg/ml using a standard curve of known quantities of cytokines.

*Results*

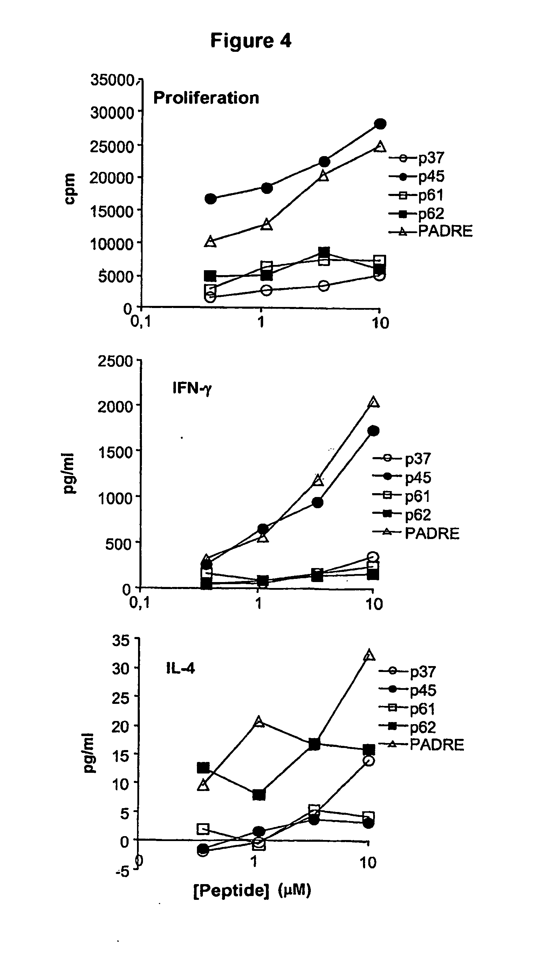

The results (Figure 4) reveal that the greatest proliferation (greater incorporation of tritiated thymidine) and production of IFN-$\gamma$ is produced in those mice immunized with the p45 and PADRE peptides. The P45 peptide considerably stimulated production of IFN-$\gamma$ and little or nothing the production of IL-4, whilst the PADRE peptide stimulated both the production of IFN-γ and IL-4. These observations permit concluding that for the HLA-DR4 restriction, p45 and PADRE induce T-helper responses corresponding to profiles of the Th1 and Th0 cytokines respectively. The p37, p61 and p62 peptides did not produce proliferation, or the production of IFN-γ. However, p37 and p62 gave rise to the production of IL-4.

**Example 4. Induction of cytotoxic T responses (CT).**

In order to study the peptides' capacity to collaborate in the induction of CT effector responses, mice (transgenic for HLA-DR4) were immunized with p37, p45, p61, p62 or with the PADRE control peptide, together with the SIINFEKL peptide [OVA(257-264)]. SIINFEKL is a cytotoxic T determinant (CTd) which binds to the class I H-2 $K^b$ molecule.

*Immunization and measurement of lysis*

To induce cytotoxic response, two mice of 4 to 6 weeks of age were immunized subcutaneously with 200 μl of a 1:1 emulsion of incomplete Freund's adjuvant and saline solution which contained 50 nanomoles of SIINFEKL peptide plus the peptide to be assayed as Dth in variable quantities.

The animals were sacrificed between 10 and 12 days after immunization to extract the popliteal, inguinal and periaortic lymph nodes. These nodes were homogenized with a syringe to obtain a cell suspension and were washed three times in RPMI 1640.

The cells obtained were incubated with the cytotoxic determinant SIINFEKL (10 μM) during 2 hours at 37 °C, they were washed twice and were cultured in 24-well plates at a concentration of $7.5 \times 10^6$ cells/well. Two days later, 2.5 U/ml of IL-2 were added to the culture and five days later the cytotoxic activity was measured, following the methodology described by Brunner (Brunner KT; "Quantitative assay of the lytic action of immune lymphoid cells on 51-Cr-labelled allogeneic target cells in vitro; inhibition by isoantibody and by drugs"; Immunology, 1968; 14:181).

The cytotoxic activity was assayed by the measurement of the release of $^{51}$Cr from the target cells, previously labelled. The target cells used were timon cells(H-2$^b$) EI-4 (Reference ATCC: TIB-39). For their labelling, 50 μCi of $^{51}$CrO$_4$Na$_2$ were added for each $10^6$ target cells in a final volume of 100 μl and they were incubated in the absence or presence of SIINFEKL peptide (at a concentration of 10 μM) during 2 hours at 37 °C. After three washes in RPMI 1640, they were resuspended in 1 ml of MC. The assay was performed in 96-well plates with U-shaped bottoms. The effector cells and the target cells were added separately (3000 per well). Different proportions of effector cells were assayed with respect to the target cells, in serial dilutions (100, 33, 11 and 3). Each assay was performed in triplicate. The final volume of each well was 200 μl.

The plates were incubated during 4 hours at 37 °C. Then, 50 μl of supernatant was extracted from each well and the radioactivity was counted in a scintillation counter.

The percentage of specific lysis was calculated according to the following formula:

$$\% \text{ Specific lysis} = 100 \times ((cpm_{experimental} - cpm_{spontaneous}) / (cpm_{maximum} - cpm_{spontaneous})$$

The maximum lysis was determined measuring the cpm (counts per minute) of 3000 target cells incubated with 5% Triton X-100 and the spontaneous lysis from cells incubated in the absence of effector cells.

The percentage of lysis indicated corresponds to the net lysis: value of the lysis against the immunized animal cells to which the lysis substrate observed against the animals cells without immunization.

*Results*

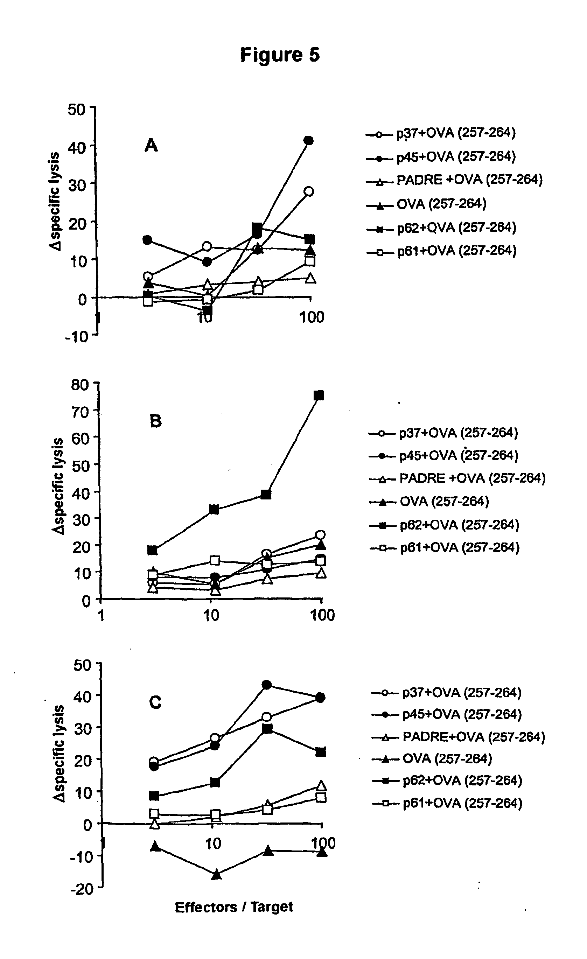

The results, represented in Figure 5, show that all peptides minus p61 and PADRE provide Th collaboration for the induction of specific SIINFEKL CT lymphocytes. Furthermore, it was possible to observe a dose-response effect so that each peptide acts better at a different dose.

**Example 5. Induction of T-helper responses *in vitro* in donors.**

In order to determine if the p37, p45 and p62 peptides could be recognized by the human Th lymphocytes of a varied population, experiments were performed with mononuclear cells of peripheral blood extracted from the umbilical cords from donors.

The extracted cells were purified using the Ficoll method (Noble PB, Cutts JH, Carroll, KK; Ficoll flotation for the separation of blood leukocyte types; Blood, 1968; 31:66-73). Once purified, the cells ($3 \times 10^6$ cells/ml) were pulsed for two hours with 10 μM of the peptide under study. The cells pulsed were washed and plated ($10^5$ cells/well) in flat-bottomed 96-well plates. On days 3 and 7, IL-2 was added. Fifteen days later, the cells of each well were subdivided in two, to contrast them respectively to cells ($10^5$ cells/well) treated with mitomicyn C, with or without each one of the p37, p45, p62 or PADRE peptides. After two days, 50 $\mu$l of each supernatant was collected and was kept frozen at -20 °C until the time at which the quantity of IFN-$\gamma$ was quantified by ELISA. The cells were pulsed on the third day, during 18 hours with 0.5 $\mu$Ci of tritiated thymidine. They were then harvested and the incorporation of thymidine was measured in a scintillation counter.

*HLA-DR typing of donors*

First, the DNA was extracted from mononuclear cells of peripheral blood from each donor. The QIAmp DNA Mini Kit (Qiagen, Valencia, USA) was used and the protocol indicated by the manufacture was followed.

For the embodiment of the typing from extracted DNA, the Inno-Lipa HLA-DRB1 Plus kit (Innogenetics, Ghent, Belgium) was used, following the protocol indicated by the manufacturer.

*Results*

Table 3 indicates the number of positive wells for each peptide and donor. Only those wells that showed a stimulation index equal to or greater than 3 were considered positive. The stimulation index (SI) was expressed as the quotient between the counts per minute between the well with peptide and the well without peptide.

**Table 3. Recognition of peptides by lymphocytes of human donors. The maximum** number of possible positive wells was 48 per peptide and donor. N indicates the total positive wells against each peptide, taking the 16 donors.

| Donors | No. of wells positive for each peptide | | | |
|---|---|---|---|---|
| No. Molecular typing | p37 | p45 | p62 | PADRE |
| 1 DRB1*03 DRB1*04 | 4 | 22 | 11 | 4 |
| 2 | 7 | 31 | 6 | 34 |
| 3 DRB1*01 DRB1*03 | 7 | 5 | 1 | 3 |
| 4 DRB1*03 DRB1*13 / DRB1*03 DRB1*15 | - | 1 | - | 1 |
| 5 DRB1*01 DRB1*08 | 4 | 3 | 14 | 8 |
| 6 DRB1*07 DRB1*011 | 1 | - | - | - |
| 7 DRB1*07 DRB1* 10 | 2 | - | - | 1 |
| 8 DRB1*01 DRB1*03 | - | - | - | 1 |
| 9 DRB1*07 DRB1*11 | 1 | - | - | - |
| 10 DRB1*04 DRB1*13 / DRB1*04 DRB1*14 | 1 | - | 3 | 1 |
| 11 | - | 1 | - | - |
| 12 DRB1*01 DRB1*04 | 1 | - | - | - |
| 13 DRB1*01 DRB1*15 | - | 1 | 1 | 1 |
| 14 DRB1*01 DRB1*13/DRB1*01 DRB1*14 | 2 | 1 | 1 | 2 |
| 15 DRB1*03 DRB1*07 | - | - | 2 | 5 |
| 16 | 1 | - | 8 | 4 |
| | **N=31** | **N=65** | **N=47** | **N=65** |

From table 3, it can be gathered that:

- the p45 and PADRE peptides were the best recognized by the lymphocytes of 16 donors; and that

- all are recognized by at least 50% of individuals.

SEQUENCE LISTING

<110> PROYECTO DE BIOMEDICINA CIMA S.L.
<120> NOVEL T-HELPER ANTIGENIC DETERMINANT (THD) PEPTIDES
<130> 1.2008.0281/ZAR
<140> 06841747.6
<141> 2008-07-22
<150> PCT/ES2006/000695
<151> 2006-12-19
<150> SPAIN P200503169
<151> 2005-12-23
<160> 25
<170> PatentIn version 3.1
<210> 1
<211> 13
<212> PRT
<213> Artificial Sequence
<220>
<223> Synthetic construct; chimeric Peptide
<400> 1

```
Phe Lys Tyr Arg Met Met Met Arg Met Arg Ala Ala Ala
1               5                   10
```

<210> 2
<211> 9
<212> PRT
<213> Artificial sequence
<220>
<223> Synthetic Construct; Chimeric Peptide
<400> 2

```
Tyr Arg Met Met Met Arg Met Arg Ala
1               5
```

<210> 3
<211> 8
<212> PRT
<213> Artificial Sequence
<220>
<223> Synthetic Construct; Chimeric Peptide
<400> 3

```
Tyr Arg Met Met Met Arg Met Arg
1               5
```

<210> 4
<211> 13
<212> PRT
<213> Artificial Sequence
<220>
<223> Synthetic Construct; chimeric Peptide
<400> 4

```
Phe Arg Tyr Arg Met Met Met Arg Met Arg Ala Ala Ala
1               5               10
```

<210> 5
<211> 13
<212> PRT
<213> Artificial sequence
<220>
<223> Synthetic Construct; Chimeric Peptide
<400> 5

```
Tyr Arg Tyr Arg Met Met Met Arg Met Arg Ala Ala Ala
1               5               10
```

<210> 6
<211> 13
<212> PRT
<213> Artificial Sequence
<220>
<223> Synthetic construct; Chimeric Peptide
<400> 6

```
Phe Lys Tyr Arg Trp Met Met Arg Trp Arg Ala·Ala Ala
1               5               10
```

<210> 7
<211> 13
<212> PRT
<213> Artificial Sequence
<220>
<223> Synthetic Construct; Chimeric Peptide
<400> 7

```
Phe Lys Tyr Arg Arg Met Met Arg Lys Arg Ala Ala Ala
1               5               10
```

<210> 8
<211> 9
<212> PRT
<213> Artificial Sequence
<220>
<223> Synthetic Construct; chimeric Peptide
<400> 8

```
Tyr Arg Ala Met Arg Ala Met Arg Ala
1               5
```

<210> 9
<211> 8
<212> PRT
<213> Artificial sequence

<220>
<223> Synthetic Construct; Chimeric Peptide
<400> 9

```
Tyr Arg Ala Met Arg Ala Met Arg
1               5
```

<210> 10
<211> 13
<212> PRT
<213> Artificial Sequence
<220>
<223> Synthetic construct; Chimeric Peptide
<400> 10

```
Phe Lys Tyr Arg Met Met Met Ala Pro Met Ala Ala Ala
1               5                   10
```

<210> 11
<211> 13
<212> PRT
<213> Artificial sequence
<220>
<223> Synthetic Construct; chimeric Peptide
<400> 11

```
Phe Lys Tyr Arg Ala Met Arg Ala Met Arg Ala Ala Ala
1               5                   10
```

<210> 12
<211> 13
<212> PRT
<213> Artificial sequence
<220>
<223> Synthetic Construct; Chimeric Peptide
<400> 12

```
Phe Lys Tyr Arg Ala Met Arg Cys Met Arg Ala Ala Ala
1               5                   10
```

<210> 13
<211> 13
<212> PRT
<213> Artificial Sequence
<220>
<223> Synthetic Construct; Chimeric Peptide
<400> 13

```
Phe Lys Tyr Arg Ala Met Arg Arg Arg Arg Ala Ala Ala
1               5                   10
```

<210> 14
<211> 13
<212> PRT
<213> Artificial Sequence
<220>
<223> Synthetic Construct; chimeric Peptide
<400> 14

```
Phe Lys Tyr Arg Arg Met Arg Arg Arg Arg Ala Ala Ala
1               5                   10
```

<210> 15
<211> 13
<212> PRT
<213> Artificial Sequence
<220>
<223> Synthetic Construct; Chimeric Peptide
<400> 15

```
Phe Lys Tyr Arg Trp Met Arg Ala Met Arg Ala Ala Ala
1               5                   10
```

<210> 16
<211> 13
<212> PRT
<213> Artificial Sequence
<220>
<223> Synthetic construct; Chimeric Peptide
<400> 16

```
Phe Lys Tyr Arg Gln Met Met Ala Pro His Ala Ala Ala
1               5                   10
```

<210> 17
<211> 13
<212> PRT
<213> Artificial sequence
<220>
<223> Synthetic Construct; chimeric Peptide
<400> 17

```
Phe Lys Tyr Arg Ala Met Arg Arg Arg His Ala Ala Ala
1               5                   10
```

<210> 18
<211> 9
<212> PRT
<213> Artificial Sequence
<220>
<223> Synthetic Construct; Chimeric Peptide
<400> 18

```
Tyr Arg Gln Met Met Ala Pro His Ala
1               5
```

<210> 19
<211> 9
<212> PRT
<213> Artificial Sequence
<220>
<223> Synthetic Construct; chimeric Peptide
<400> 19

```
Tyr Arg Ala Met Arg Arg Arg His Ala
1               5
```

<210> 20
<211> 13
<212> PRT
<213> Artificial Sequence
<220>
<223> Synthetic Construct; Chimeric Peptide
<400> 20

```
Phe Lys Tyr Tyr Ala Met Arg Cys Met Arg Ala Ala Ala
1               5                   10
```

<210> 21
<211> 13
<212> PRT
<213> Artificial Sequence
<220>
<223> Synthetic Construct; Chimeric Peptide
<400> 21

```
Phe Lys Tyr His Gln Met Met Ala Pro His Ala Ala Ala
1               5                   10
```

<210> 22
<211> 13
<212> PRT
<213> Artificial Sequence
<220>
<223> Synthetic Construct; Chimeric Peptide
<400> 22

```
Phe Lys Tyr Arg Trp Val Arg Ala Leu Arg Ala Ala Ala
1               5                   10
```

<210> 23
<211> 13
<212> PRT

<213> Artificial sequence
<220>
<223> Synthetic Construct; Chimeric Peptide
<400> 23

```
Ala Lys Phe Val Ala Ala Trp Thr Leu Lys Ala Ala Ala
1               5                   10
```

<210> 24
<211> 15
<212> PRT
<213> Artificial Sequence
<220>
<223> Synthetic Construct; Synthetic Peptide
<400> 24

```
Ala Pro Lys Tyr Val Lys Gln Asn Thr Leu Lys Leu Ala Thr Gly
1               5                   10                  15
```

<210> 25
<211> 8
<212> PRT
<213> Artificial Sequence
<220>
<223> Synthetic Construct; Synthetic Peptide <400> 25

```
Ser Ile Ile Asn Phe Glu Lys Leu
1               5
```

## Claims

1. A chimeric peptide with capacity to bind to at least one allelic form of the HLA-DR molecule, **characterized in that** its sequence of amino acids consists of the formula selected from:

   a) $a_1$-$a_2$-Y-$a_4$-$a_5$-$a_6$-$a_7$-$a_8$-$a_9$-$a_{10}$-A-A-A; and
   b) the SEQ. ID. NO: 21;
   wherein Y is Tyr; A is Ala; $a_1$ is Phe or Tyr; $a_2$ is Lys or Arg; if $a_6$ is Met and $a_{10}$ is Arg, then $a_4$ is any of the 20 natural amino acids, else $a_4$ is Arg; $a_5$, $a_7$ and $a_9$ are any of the 20 natural amino acids; if $a_4$ and $a_{10}$ are Arg, then $a_6$ is any of the 20 natural amino acids, else $a_6$ is Met; if $a_4$ is Arg, Tyr or His, $a_6$ is Met or Val, and $a_{10}$ is Met, His or Arg, then $a_8$ is any of the 20 natural amino acids, else $a_8$ is Arg; and if $a_4$ is Arg or His, and $a_6$ is Met, then $a_{10}$ is any of the 20 natural amino acids, else $a_{10}$ is Arg.

2. A chimeric peptide with capacity to bind to at least one allelic form of the HLA-DR molecule according to claim 1, **characterized in that** its formula is selected from:

   I) $a_1$-$a_2$Y-R-$a_5$-M-$a_7$R-$a_9$-R-A-A-A;
   II) $a_1$-$a_2$-Y-R-$a_5$-M-$a_7$-$a_8$-$a_9$-$a_{10}$-A-A-A;
   III) $a_1$-$a_2$-Y-$a_4$-$a_5$-M-$a_7$-$a_8$-$a_9$-R-A-A-A; and
   IV) $a_1$-$a_2$-Y-R-$a_5$-$a_6$-$a_7$-$a_8$-$a_9$-R-A-A-A;
   where Y is Tyr; R is Arg; M is Met; A is Ala; $a_1$ is Phe or Tyr; $a_2$ is Lys or Arg; $a_4$ is any of the 20 natural amino acids other than Arg; $a_5$, $a_7$ and $a_9$ are any of the 20 natural amino acids; $a_6$ is any of the 20 natural amino acids other than Met; $a_8$ is any of the 20 natural amino acids other than Arg; and $a_{10}$ is any of the 20 natural amino acids other than Arg.

3. A peptide according to one of claims 1 or 2, where said HLA-DR allelic form is selected from: HLA-DR1, HLA-DR2, HLA-DR3, HLA-DR4, HLA-DR7, HLA-DR8 or HLA-DR11.

4. A peptide according to one of claims 1 to 3, **characterized in that** it binds to at least 2 allelic forms of HLA-DR.

5. A peptide according to one of claims 1 to 4, **characterized in that** it induces the activation of T-helper cells (Th).

6. A peptide according to one of claims 1 to 5, **characterized in that** it induces the activation of cytotoxic T cells (CT).

7. A peptide according to one of claims 1 to 6, **characterized in that** it has a sequence selected from SEQ. ID. NO: 1, SEQ. ID. NO: 4, SEQ. ID. NO: 5, SEQ. ID. NO: 6, SEQ. ID. NO: 7, SEQ. ID. NO: 10, SEQ. ID. NO: 11, SEQ. ID. NO: 12, SEQ. ID. NO: 13, SEQ. ID. NO: 14, SEQ. ID. NO: 15, SEQ. ID. NO: 16, SEQ. ID. NO: 17, SEQ. ID. NO: 20 and SEQ. ID. NO: 22.

8. A pharmaceutical composition **characterized in that** it comprises at least one peptide described in one of claims 1 to 7 and a pharmaceutically acceptable excipient.

9. A pharmaceutical composition according to claim 8, **characterized in that** it further comprises another immunogen.

10. A pharmaceutical composition according to one of claims 8 or 9, **characterized in that** it comprises a cytotoxic T determinant (CTd) and a T-helper determinant (Thd), where the determinant Thd is a peptide described in one of claims I to 7.

11. Use of a peptide described in one of claims I to 7 in the preparation of a pharmaceutical composition useful to stimulate the immune response.

12. Use of a peptide according to claim 11, **characterized in that** said pharmaceutical composition is useful for inducing the activation of T-helper cells Th (Th1, Th2 or Th0).

13. Use of a peptide according to claim 11, **characterized in that** said pharmaceutical composition is useful for inducing a cytotoxic T immune response (CTL).

14. Use of a peptide according to claim 11, **characterized in that** said pharmaceutical composition is useful for inducing a humoral immune response.


**Patentansprüche**

1. Chimäres Peptid mit der Fähigkeit, an mindestens eine allelische Form des HLA-DR-Moleküls zu binden, **dadurch gekennzeichnet, dass** seine Sequenz von Aminosäuren aus der Formel, ausgewählt aus:

   a) $a_1$-$a_2$-Y-$a_4$-$a_5$-$a_6$-$a_7$-$a_8$-$a_9$-a10-A-A-A; und
   b) der SEQ ID Nr. 21
   besteht, worin Y für Tyr steht; A für Ala steht; $a_1$ für Phe oder Tyr steht; $a_2$ für Lys oder Arg steht; falls $a_6$ für Met steht und $a_{10}$ für Arg steht, dann $a_4$ für eine der 20 natürlichen Aminosäuren steht, sonst $a_4$ für Arg steht; $a_5$, $a_7$ und $a_9$ für eine der 20 natürlichen Aminosäuren stehen; falls $a_4$ und $a_{10}$ für Arg stehen, dann $a_6$ für eine der 20 natürlichen Aminosäuren steht, sonst $a_6$ für Met steht; falls $a_4$ für Arg, Tyr oder His steht, $a_6$ für Met oder Val steht und $a_{10}$ für Met, His oder Arg steht, dann $a_8$ für eine der 20 natürlichen Aminosäuren steht, sonst $a_8$ für Arg steht; und falls $a_4$ für Arg oder His steht und $a_6$ für Met steht, dann $a_{10}$ für eine der 20 natürlichen Aminosäuren steht, sonst $a_{10}$ für Arg steht.

2. Chimäres Peptid mit der Fähigkeit, an mindestens eine allelische Form des HLA-DR-Moleküls zu binden, gemäß Anspruch 1, **dadurch gekennzeichnet, dass** seine Formel ausgewählt ist aus:

   I) $a_1$-$a_2$-Y-R-$a_5$-M-$a_7$-R-$a_9$-R-A-A-A;
   II) $a_1$-$a_2$-Y-R-$a_5$-M-$a_7$-$a_8$-$a_9$-$a_{10}$-A-A-A;
   III) $a_1$-$a_2$-Y-$a_4$-$a_5$-M-$a_7$-$a_8$-$a_9$-R-A-A-A; und
   IV) $a_1$-$a_2$-Y-R-$a_5$-$a_6$-$a_7$-$a_8$-$a_9$-R-A-A-A;

worin Y für Tyr steht; R für Arg steht; M für Met steht; A für Ala steht; $a_1$ für Phe oder Tyr steht; $a_2$ für Lys oder Arg steht; $a_4$ für eine andere der 20 natürlichen Aminosäuren als Arg steht; $a_5$, $a_7$ und $a_9$ für eine der 20 natürlichen Aminosäuren stehen; $a_6$ für eine andere der 20 natürlichen Aminosäuren als Met steht; $a_8$ für eine andere der 20 natürlichen Aminosäuren als Arg steht; und $a_{10}$ für eine andere der 20 natürlichen Aminosäuren als Arg steht.

3. Peptid gemäß einem von Ansprüchen 1 oder 2, worin besagte HLA-DR allelische Form ausgewählt ist aus: HLA-DR1, HLA-DR2, HLA-DR3, HLA-DR4, HHLA-DR7, HLA-DR8 oder HLA-DR11.

4. Peptid gemäß einem von Ansprüchen 1 bis 3, **dadurch gekennzeichnet, dass** es an mindestens 2 allelische Formen von HLA-DR bindet.

5. Peptid gemäß einem von Ansprüchen 1 bis 4, **dadurch gekennzeichnet, dass** es die Aktivierung von T-Helferzellen (Th) induziert.

6. Peptid gemäß einem von Ansprüchen 1 bis 5, **dadurch gekennzeichnet, dass** es die Aktivierung von zytotoxischen T-Zellen (CT) induziert.

7. Peptid gemäß einem von Ansprüchen 1 bis 6, **dadurch gekennzeichnet, dass** es eine Sequenz hat, ausgewählt aus SEQ ID Nr. 1, SEQ ID Nr. 4, SEQ ID Nr. 5, SEQ ID Nr. 6, SEQ ID Nr. 7, SEQ ID Nr. 10, SEQ ID Nr. 11, SEQ ID Nr. 12, SEQ ID Nr. 13, SEQ ID Nr. 14, SEQ ID Nr. 15, SEQ ID Nr. 16, SEQ ID Nr. 17, SEQ ID Nr. 20 und SEQ ID Nr. 22.

8. Pharmazeutische Zusammensetzung, **dadurch gekennzeichnet, dass** sie mindestens ein in einem von Ansprüchen 1 bis 7 beschriebenes Peptid und einen pharmazeutisch annehmbaren Arzneimittelträger umfasst.

9. Pharmazeutische Zusammensetzung gemäß Anspruch 8, **dadurch gekennzeichnet, dass** sie ferner ein weiteres Immunogen umfasst.

10. Pharmazeutische Zusammensetzung gemäß einem von Ansprüchen 8 oder 9, **dadurch gekennzeichnet, dass** sie eine zytotoxische T-Determinante (CTd) und eine T-Helfer-Determinante (Thd) umfasst, wobei die Determinante Thd ein in einem von Ansprüchen 1 bis 7 beschriebenes Peptid ist.

11. Verwendung eines in einem von Ansprüchen 1 bis 7 beschriebenen Peptids in der Herstellung einer pharmazeutischen Zusammensetzung, welche brauchbar ist, die Immunantwort zu stimulieren.

12. Verwendung eines Peptids gemäß Anspruch 11, **dadurch gekennzeichnet, dass** besagte pharmazeutische Zusammensetzung brauchbar zum Induzieren der Aktivierung von T-Helferzellen Th (Th1, Th2 oder Th0) ist.

13. Verwendung eines Peptids gemäß Anspruch 11, **dadurch gekennzeichnet, dass** besagte pharmazeutische Zusammensetzung brauchbar zum Induzieren einer zytotoxischen T-Immunantwort (CTL) ist.

14. Verwendung eines Peptids gemäß Anspruch 11, **dadurch gekennzeichnet, dass** besagte pharmazeutische Zusammensetzung brauchbar zum Induzieren einer humoralen Immunantwort ist.

## Revendications

1. Peptide chimère ayant la capacité de se lier au moins à une forme allélique de la molécule HLA-DR, **caractérisé en ce que** sa séquence d'acides aminés est constituée de la formule choisie parmi :

a) $a_1$-$a_2$-Y-$a_4$-$a_5$-$a_6$-$a_7$-$a_8$-$a_9$-$a_{10}$-A-A-A ; et
b) la SEQ. ID. NO:21 ;
où Y est Tyr ; A est Ala ; $a_1$ est Phe ou Tyr ; $a_2$ est Lys ou Arg ; si $a_6$ est Met et $a_{10}$ est Arg, alors $a_4$ est n'importe lequel des 20 acides aminés naturels, sinon $a_4$ est Arg ; $a_5$, $a_7$ et $a_9$ sont n'importe lequel des 20 acides aminés naturels ; si $a_4$ et $a_{10}$ sont Arg, alors $a_6$ est n'importe lequel des 20 acides aminés naturels, sinon $a_6$ est Met ; si $a_4$ est Arg, Tyr ou His, $a_6$ est Met ou Val, et $a_{10}$ est Met, His ou Arg, alors $a_8$ est n'importe lequel des 20 acides aminés naturels, sinon $a_8$ est Arg ; et si $a_4$ est Arg ou His, et $a_6$ est Met, alors $a_{10}$ est n'importe lequel des 20 acides aminés naturels, sinon $a_{10}$ est Arg.

2. Peptide chimère ayant la capacité de se lier à au moins une forme allélique de la molécule HLA-DR selon la revendication 1, **caractérisé en ce que** sa formule est choisie parmi :

I) $a_1$-$a_2$-Y-R-$a_5$-M-$a_7$-R-$a_9$-R-A-A-A ;
II) $a_1$-$a_2$-Y-R-$a_5$-M-$a_7$-$a_S$-$a_9$-$a_{10}$-A-A-A ;
III) $a_1$-$a_2$-Y-$a_4$-$a_5$-M-$a_7$-$a_8$-$a_9$-R-A-A-A ; et
IV) $a_1$-$a_2$-Y-R-$a_5$-$a_6$-$a_7$-$a_8$-$a_9$-R-A-A-A ;
où Y est Tyr ; R est Arg ; M est Met ; A est Ala ; $a_1$ est Phe ou Tyr ; $a_2$ est Lys ou Arg ; $a_4$ est n'importe lequel des 20 acides aminés naturels autre que Arg ; $a_5$, $a_7$ et $a_9$ sont n'importe lesquels des 20 acides aminés naturels; $a_6$ est n'importe lequel des 20 acides aminés naturels autre que Met ; $a_8$ est n'importe lequel des 20 acides aminés naturels autre que Arg ; et $a_{10}$ est n'importe lequel des 20 acides aminés naturels autre que Arg.

3. Peptide selon l'une des revendications 1 ou 2, où ladite forme allélique de HLA-DR est choisie parmi : HLA-DR1, HLA-DR2, HLA-DR3, HLA-DR4, HLA-DR7, HLA-DR8 ou HLA-DR11.

4. Peptide selon l'une des revendications 1 à 3, **caractérisé en ce qu'**il se lie à au moins 2 formes alléliques de HLA-DR.

5. Peptide selon l'une des revendications 1 à 4, **caractérisé en ce qu'**il induit l'activation des cellules T-auxiliaires (Th).

6. Peptide selon l'une des revendications 1 à 5, **caractérisé en ce qu'**il induit l'activation des cellules T cytotoxiques (CT).

7. Peptide selon l'une des revendications 1 à 6, **caractérisé en ce qu'**il a une séquence choisie parmi SEQ. ID. NO: 1, SEQ. ID. NO:4, SEQ. ID. NO:5, SEQ. ID. NO:6, SEQ. ID. NO:7, SEQ. ID. NO:10, SEQ. ID. NO:11, SEQ. ID. NO: 12, SEQ. ID. NO:13, SEQ. ID. NO:14, SEQ. ID. NO:15, SEQ. ID. NO:16, SEQ. ID. NO:17, SEQ. ID. NO:20 et SEQ. ID. NO:22.

8. Composition pharmaceutique **caractérisée en ce qu'**elle comprend au moins un peptide décrit dans l'une des revendications 1 à 7 et un excipient pharmaceutiquement acceptable.

9. Composition pharmaceutique selon la revendication 8, **caractérisée en ce qu'**elle comprend en plus un autre immunogène.

10. Composition pharmaceutique selon l'une des revendications 8 ou 9, **caractérisée en qu'**elle comprend un déterminant T cytotoxique (CTd) et un déterminant T-auxiliaire (Thd), où le déterminant Thd est un peptide décrit dans une des revendications 1 à 7.

11. Utilisation d'un peptide décrit dans une des revendications 1 à 7 dans la préparation d'une composition pharmaceutique utile pour stimuler la réponse immune.

12. Utilisation d'un peptide selon la revendication 11, **caractérisée en ce que** ladite composition pharmaceutique est utile pour induire l'activation des cellules T-auxiliaires Th (Th1, Th2 ou Th0).

13. Utilisation d'un peptide selon la revendication 11, **caractérisée en ce que** ladite composition pharmaceutique est utile pour induire une réponse immune T cytotoxique (CTL).

14. Utilisation d'un peptide selon la revendication 11, **caractérisée en ce que** ladite composition pharmaceutique est utile pour induire une réponse immune humorale.

EP 1 978 026 B1

## Figure 1

% Relative binding (% $B_R$) :

- □ <25
- 25-50
- 50-75
- ■ 75-100
- ■ >100

## Figure 2

## Figure 3

# Figure 4

# Figure 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 9507707 A
- WO 9832456 A
- EP 0876398 A
- ES 06841747
- ES 2006000695 W
- ES P200503169

### Non-patent literature cited in the description

- The immune system in health and disease. Immunobiology. Current Biology Ltd / Garland Publishing Inc, 1997
- **Alexander J et al.** *Immunity,* 1994, vol. 1, 751-761
- **Borrás-Cuesta F. et al.** Specific and general HLA-DR binding motifs: comparison algorithms. *Human Immunol.,* 2000, vol. 61, 266-278
- **Chicz R.M. et al.** Predominant naturally processed peptides bound to HLA-DR1 are derived from MHC-related molecules and are heterogeneous in size. *Nature,* 1992, vol. 358, 764-768
- The current protocols in protein chemistry. John Wiley & Sons, 01 May 2005
- **GT Hermanson ; AK Mallia ; PK Smith.** Immobilized affinity ligand Techniques. Academic Press, Inc, 1992
- Tecnologia farmaceutica. **J.L. Vila Jato.** Synthesis. 1997, vol. I,II
- **S.K. Niazi.** Handbook of pharmaceutical manufacturing formulations. CRC Press, 2004, vol. I-VI
- **Merrifield RB.** Solid phase synthesis. *I. J Am Chem Soc,* 1963, vol. 85, 2149
- **Atherton E.** Procedures for solid phase synthesis. *J Chem Soc Perkin Trans,* 1989, vol. 1, 538
- **Kaiser.** Color test for detection of free terminal amino groups in the solid-phase synthesis of peptides. *Anal Biochem.,* 1970, vol. 34, 595-598
- **Busch R ; Rothbard J.** Degenerate binding of immunogenic peptides to HLA-DR proteins on B cell surfaces. *Int Immunol,* 1990, vol. 144, 1849
- **Rothbard JB.** Degenerate binding of immmunogenic peptides. *Int Immunol,* 1990, vol. 2, 443-451
- **Brunner KT.** Quantitative assay of the lytic action of immune lymphoid cells on 51-Cr-labelled allogeneic target cells in vitro; inhibition by isoantibody and by drugs. *Immunology,* 1968, vol. 14, 181
- **Noble PB ; Cutts JH ; Carroll, KK.** Ficoll flotation for the separation of blood leukocyte types. *Blood,* 1968, vol. 31, 66-73